Europäisches Patentamt

European Patent Office

Office européen des brevets

(19)

(11) Numéro de publication: **0 240 480**
**B1**

(12) **FASCICULE DE BREVET EUROPEEN**

(45) Date de publication du fascicule du brevet:
**23.05.90**

(21) Numéro de dépôt: **87870009.5**

(22) Date de dépôt: **23.01.87**

(51) Int. Cl.⁵: **C07C 2/12**, C07C 5/27,
C07C 5/41, C10G 35/095,
B01J 29/02

(54) **Procédé de traitement d'hydrocarbures avec des catalyseurs de type silicalite ou TEA-silicate stabilisés par halogénation.**

(30) Priorité: **29.01.86 LU 86277**

(43) Date de publication de la demande:
**07.10.87 Bulletin 87/41**

(45) Mention de la délivrance du brevet:
**23.05.90 Bulletin 90/21**

(84) Etats contractants désignés:
**AT BE CH DE ES FR GB IT LI NL SE**

(56) Documents cités:
**EP-A- 0 201 856**
**EP-A- 0 235 110**
**EP-A- 0 239 557**
**GB-A- 2 106 132**
**GB-A- 2 118 570**
**GB-A- 2 144 447**

(73) Titulaire: **FINA RESEARCH S.A., Zone Industrielle C,
B-6520 Seneffe(BE)**

(72) Inventeur: **Debras, Guy L.G., 176 avenue Joseph Abras,
B-5001 Belgrade(BE)**
Inventeur: **Cahen, Raymond M., 21 avenue Commandant
Lothaire, B-1040 Bruxelles(BE)**
Inventeur: **De Clippeleir, Georges E.M.J., 5, Petrus
Huysegomstraat, B-1600 Sint-Pieters-Leeuw(BE)**

(74) Mandataire: **Leyder, Francis, c/o Fina Research S.A.
Zone Industrielle C, B-6520 Feluy(BE)**

**Description**

La présente invention se rapporte à un procédé de traitement catalytique qui se déroule sous pression et en présence de TEA-silicate ou de silicalite comme catalyseur. La présente invention se rapporte en particulier à un procédé de traitement catalytique sous pression en présence de catalyseur silicalite ou TEA-silicate et qui nécessite des régénérations ou des remplacements de catalyseur beaucoup moins fréquents.

Il est bien connu que les catalyseurs à base de silicalite ou TEA-silicate sont relativement stables lorsqu'ils sont utilisés dans des procédés de traitement catalytique qui se déroulent à la pression atmosphérique. C'est la raison pour laquelle on a déjà utilisé la silicalite ou le TEA-silicate seuls dans des procédés comme l'isomérisation des hydrocarbures, la dimérisation des oléfines, l'aromatisation et le réformage de charges paraffiniques et plus généralement la conversion des hydrocarbures, pour autant que l'on réalise le procédé à la pression atmosphérique.

Or, il est généralement intéressant de réaliser ces procédés à des pressions plus élevées que la pression atmosphérique.

Pour pouvoir opérer à pressions plus élevées tout en conservant une certaine stabilité et activité au catalyseur de type silicalite, on a déjà proposé dans le brevet US 4.414.423 d'incorporer un métal du Groupe II B du Tableau Périodique des Eléments, en général du zinc ou du cadmium. L'incorporation peut être réalisée par toute méthode connue, comme l'imprégnation et autres techniques usuelles. Cette technique a été utilisée dans un procédé de traitement catalytique contenant des oléfines gazeuses. Il faut cependant noter que malgré la tentative pour stabiliser la silicalite, on observe une diminution importante du rendement après un temps de fonctionnement relativement court.

Selon le brevet US 4.414.423, on a également proposé de réduire la vitesse spatiale horaire de la charge afin de pallier en partie la réduction d'activité. Cependant, l'homme de l'art sait parfaitement qu'en réduisant la vitesse spatiale horaire de la charge, il faut nécessairement agrandir les installations pour maintenir la même productivité.

Il y a donc un besoin pour posséder un procédé de traitement catalytique sous pression à base de catalyseur de type silicalite ou de type TEA-silicate qui permette de réduire considérablement le nombre de régénérations ou de remplacements du catalyseur.

La présente invention a pour objet un procédé de traitement catalytique sous pression en présence de silicalite ou de TEA-silicate comme catalyseur qui permette d'utiliser le catalyseur pendant une plus longue période de temps.

La présente invention a également pour objet un procédé de traitement catalytique sous pression en présence d'un catalyseur du type silicalite ou TEA-silicate qui a une longue activité et qui est efficace pendant de longues périodes de temps sans nécessiter de régénération ou de remplacement.

Le procédé de la présente invention pour le traitement catalytique sous pression en présence de silicalite ou de TEA-silicate comme catalyseur, est caractérisé en ce qu'il consiste à utiliser une silicalite ou un TEA-silicate préalablement halogénés et à effectuer le traitement en présence d'eau.

Le procédé de la présente invention est particulièrement applicable à la conversion d'oléfines, à leur dimérisation, ainsi qu'à l'aromatisation des charges paraffiniques. Les charges que l'on peut utiliser selon le procédé de l'invention sont des charges comprenant des oléfines gazeuses à température ambiante comme des oléfines en $C_2$, $C_3$, et $C_4$ ou des mélanges de celles-ci afin de les isomériser ou de les oligomériser. On peut également traiter des charges contenant des hydrocarbures paraffiniques en $C_2$, $C_3$, et $C_4$. On peut également utiliser des charges provenant des courants de raffinage ou de réformage. Ces charges sont généralement destinées à être aromatisées pour en améliorer l'indice d'octane. On peut également utiliser des charges ayant déjà subi un réformage pour autant que le traitement que l'on doive leur faire subir s'effectue sous pression.

La silicalite halogénée utilisée dans le procédé de la présente invention est obtenue tout d'abord à partir d'une silicalite, c'est-à-dire une silice cristalline polymorphe qui n'a pas de capacité d'échange par comparaison aux zéolithes. L'aluminium peut être présent dans ces catalyseurs de type silicalite, mais uniquement sous forme d'impuretés provenant des produits de départ et notamment de la source de silice utilisée; en aucun cas cette silicalite ne peut être considérée comme étant un silicate métallique. La description détaillée ainsi que les méthodes pour obtenir ces silicalites sont décrites dans le brevet US 4.061.724 de Grose.

Le TEA-silicate est un type distinct de catalyseur synthétisé à partir de systèmes de réactions essentiellement exempts de réactif contenant de l'aluminium et qui sont par conséquent entièrement exempts de tétraèdres $AlO_4^-$ ou qui ne contiennent pas, cristallographiquement, des quantités significatives de ces tétraèdres. Le TEA-silicate est décrit dans le brevet US 4.104.294. Il s'agit d'organosilicates métalliques cristallins préparés à partir d'un système $R_2O:0-8,O/M_2O: 12 - 40/SiO_2$ amorphe: 100 - 500/$H_2O$, où R représente le cation TEA ou tétraéthylammonium et M est le cation d'un métal alcalin. Il est indiqué que le TEA-silicate contient seulement le groupement organique dans sa forme synthétisée, car les parties organiques sont éliminées par calcination avant l'utilisation comme catalyseur de conversion d'hydrocarbures.

Le TEA-silicate utilisé dans le procédé de la présente invention peut être caractérisé comme des organosilicates cristallins microporeux qui sont préparés hydrothermiquement en utilisant un mélange réac-

2

tionnel comprenant des cations tétraéthylammonium, des cations de métaux alcalins, de l'eau et une source de silice réactive. Différents des zéolites cristallines, qui sont des aluminosilicates comprenant des réseaux tridimensionnels de tétraèdres $SiO_4$ et $AlO_4$, joints entre eux par les atomes d'oxygène, les organosilicates cristallins utilisés dans le procédé de la présente invention sont synthétisés à partir de systèmes réactionnels qui sont essentiellement exempts de réactifs contenant de l'aluminium. Ces TEA-silicates peuvent être préparés selon les méthodes décrites dans le brevet US 4.104.294. La teneur en aluminium de ces composés peut varier selon la quantité d'aluminium contenue comme impureté dans les matériaux de départ. Par exemple, le catalyseur TEA-silicate particulier utilisé dans le procédé de la présente invention peut avoir une teneur en aluminium légèrement plus élevée que celle qui est décrite dans le brevet US 4.104.294 due aux impuretés d'aluminium dans les produits de départ et notamment la silice amorphe solide utilisée pour sa préparation.

Ces catalyseurs sont hydrophobes et organophiles, ils absorbent le néopentane, ce qui suppose une ouverture de pore plus grande qu'environ 6,2 Å (0,62 nm).

Cependant, dans le procédé de la présente invention, qui s'effectue sous pression superatmosphérique, le TEA-silicate ou la silicalite comme tels ne conviennent pas, car ils ne sont pas suffisamment stables.

La Demanderesse a maintenant trouvé qu'en utilisant une silicalite ou un TEA-silicate halogénés, on obtenait d'excellents résultats du point de vue longévité du catalyseur, ce qui par conséquent permettait de réaliser le procédé pendant une plus longue durée, lorsque ce procédé faisait intervenir de l'eau.

La teneur en halogène dans le catalyseur dépend de plusieurs facteurs, comme par exemple le temps d'halogénation et le type d'agent halogénant. De plus, dans beaucoup de cas, plus la teneur en halogène est élevée et plus le catalyseur de type silicalite est stable. Généralement, la teneur en halogène sur la silicalite est comprise entre 0,1 et 5 % en poids et plus particulièrement entre 0,2 et 1,0 % en poids.

On peut préparer les catalyseurs halogénés en effectuant l'halogénation d'un TEA-silicate ou d'une silicalite usuels à une température comprise entre environ 200°C et environ 500°C, selon l'agent halogénant choisi; cette température sera de préférence comprise entre environ 250°C et environ 300°C lorsqu'on utilise un composé chloré ou bromé, et entre environ 450°C et environ 500°C lorsqu'on utilise un composé fluoré.

On réalise l'halogénation du catalyseur par mise en contact de celui-ci avec un courant gazeux comprenant l'agent halogénant et un véhiculeur gazeux non-réducteur. On a trouvé que ce traitement doit être réalisé dans des conditions spécifiques de façon à obtenir des catalyseurs suffisamment actifs pour des procédé catalytiques à hautes pressions.

L'une des conditions réside dans le choix de l'agent halogénant. Cet agent est de préférence un composé organique aliphatique saturé chloré, bromé ou fluoré volatil, ayant une tension de vapeur d'au moins 13 kPa à une température d'environ 200 à 230°C. Il est préférable d'utiliser un composé halogéné ayant une tension de vapeur d'environ 40 à 53 kPa ou même plus dans cet intervalle de températures.

Comme agent halogénant actif, on peut citer des composés halogénés ayant un rapport halogène/carbone supérieur ou égal à 1, contenant de 1 à 4 atomes de carbone, et pouvant contenir de l'oxygène. Comme agents halogénants particulièrement appropriés pour produire les catalyseurs de la présente invention, on peut citer les paraffines halogénées contenant de 1 à 4 atomes de carbone et les éthers halogénés contenant de 2 à 4 atomes de carbone et qui répondent aux conditions indiquées ci-dessus du point de vue volatilité et rapport halogène/carbone.. A titre d'exemples typiques de paraffines halogénées, on peut citer le tétrachlorure, le tétrabromure, ou le tétrafluorure de carbone, le chloroforme, le bromoforme, le fluoroforme, l'hexachloroéthane, l'hexafluoroéthane et le pentachloroéthane, ainsi que $CH_2F_2$. Comme autres agents halogénants, on peut encore citer le di-trichlorométhyl éther, le di-pentachloroéthyl éther et leurs homologues bromés.

Parmi les composés cités ci-dessus, le tétrachlorure de carbone et le di-trichlorométhyl éther sont particulièrement appropriés comme agents halogénants, alors que le chlore et le brome moléculaire ainsi que le chlorure d'hydrogène ou le bromure d'hydrogène sont moins efficaces.

Le véhiculeur gazeux non-réducteur que l'on peut utiliser est généralement l'azote, le dioxyde de carbone, l'oxygène ou leurs mélanges.

La quantité totale d'agent halogénant qui doit être utilisée ainsi que le temps de contact avec la silicalite ou le TEA-silicate sont également réglés de façon à produire des catalyseurs contenant environ 0,1 à environ 5 % en poids de chlore et/ou de brome et/ou de fluor. Le temps de contact nécessaire dépend de nombreux facteurs comme le nombre d'atomes d'halogène dans l'agent halogénant et la concentration en agent halogénant dans le flux gazeux ou la tension de vapeur de cet agent. En général, il est compris entre ¼ et 96 heures, plus particulièrement entre 1 et 12 heures. La teneur préférée en halogène du catalyseur dépend de nombreux facteurs. Par exemple, dans la majorité des cas, l'activité du catalyseur est d'autant plus élevée que la teneur en halogène est grande.

Cependant, on a constaté que dans la plupart des cas, il était plus intéressant d'avoir une teneur en halogène comprise entre 0,1 et 1 % en poids.

Le TEA-silicate ou la silicalite halogénés de la présente invention peuvent être produits sous forme de poudre, de granulés, d'extrudés ou même sous forme de billes. Ces particules peuvent être utilisées en lit fixe ou en lit mobile. Les catalyseurs de la présente invention peuvent être préparés in situ (auquel cas on n'est pas obligé de récupérer le catalyseur) ou ex situ. .

Mais, dans tous les cas, il est nécessaire que le procédé de traitement catalytique de la présente invention se déroule en présence d'eau.

En effet, il existe une différence essentielle entre les zéolites et le catalyseur halogéné préparé selon le procédé de l'invention: les zéolites ne supportent généralement pas la présence d'eau (qu'elle soit sous forme de liquide ou de vapeur), alors que les catalyseurs de l'invention y résistent, voire présentent une stabilité accrue.

Généralement, on introduit de l'eau avec la charge dans le réacteur, dans un rapport molaire eau/charge compris entre 0,5 et 1,5 et le plus souvent compris entre 0,5 et 1.

Les procédés de traitement catalytique de la présente invention se déroulent à une température généralement comprise entre 250 et 550°C, le plus souvent entre 300 et 500°C.

On réalise le procédé de traitement catalytique à une pression comprise entre $10^5$ et $7.10^6$ Pa.

Plus spécifiquement, lorsque l'on réalise un procédé de dimérisation de charge d'oléfines en $C_4$ on utilise des conditions opératoires telles que la température soit comprise entre 300 et 500°C et la pression entre $2.10^5$ et $6.10^6$ Pa et une quantité d'eau telle que le rapport molaire eau/charge soit compris entre 0,6 et 0,9. On réalise le procédé de l'invention à des vitesses spatiales liquides horaires relativement élevées et qui sont comprises entre 5 et 200. Elles dépendent cependant du type de charges à traiter, de la température dans le réacteur et du type de produit désiré.

Bien que l'on ne connaisse pas les raisons de l'efficacité du procédé de traitement catalytique sous pression de la présente invention, on a trouvé qu'il était particulièrement approprié pour la dimérisation et l'isomérisation des charges d'hydrocarbures contenant des oléfines normalement gazeuses, pour autant que l'on respecte les conditions opératoires énoncées ci-dessus. On a ainsi trouvé que les catalyseurs du type silicalite ou du type TEA-silicate halogénés avaient une activité particulièrement longue lorsqu'ils étaient utilisés en présence d'eau.

Si, malgré la stabilisation fournie par le procédé de la présente invention, on désire régénérer un catalyseur du type silicalite ou du type TEA-silicate, halogéné comme décrit ci-dessus, on peut le régénérer selon des techniques bien connues de l'homme de l'art, en brûlant les produits hydrocarbonés qui s'y sont accumulés, par exemple en les calcinant à des températures comprises entre 450°C et 550°C en présence d'azote contenant des teneurs progressivement croissantes en oxygène, jusqu'à ce que la teneur en $CO_2$ dans l'air soit inférieure à 0,1 % en volume.

Les exemples suivants sont donnés afin de mieux illustrer la présente invention, mais sans pour autant en limiter la portée.

Exemple 1

On a chargé dans un réacteur de la silicalite que l'on a chauffée à 500°C pendant 3 heures sous un courant d'azote ayant une vitesse spatiale horaire gazeuse (GHSV) de 500 (ml/ml). On a refroidi à 280°C en maintenant le débit d'azote, puis on a saturé pendant 4 heures l'azote envoyé vers le réacteur avec du $CCl_4$.

Sur la silicalite ainsi chlorée, contenant 0,8 % en poids de chlore, on a fait passer, à une température de 340°C, sous une pression de $14,7.10^5$ Pa et avec une vitesse spatiale horaire liquide (LHSV) de 30, une charge d'hydrocarbures en $C_4$ ayant la composition suivante:

| | |
|---|---|
| 53,3% (en poids) | de n-butènes |
| 1,2 | d'isobutène |
| 44,6 | de butanes |
| 0,9 | d'hydrocarbures plus légers |

ainsi que de la vapeur d'eau dans un rapport molaire eau/charge de 1/1.

On a obtenu les valeurs suivantes (exprimées en % poids) pour la conversion des n-butènes et la sélectivité en essences (hydrocarbures ayant une température d'ébullition comprise entre 36 et 200°C); on a calculé le rendement en essences (= conversion x sélectivité) :

| après | conversion | sélectivité | rendement |
|---|---|---|---|
| 9 heures | 94,6% | 86,8% | 82,1% |
| 29 " | 91,6% | 87,6% | 80,2% |
| 53 " | 90,0% | 85,4% | 76,9% |
| 73 " | 89,5% | 84,8% | 75,9% |
| 95 " | 90,1% | 81,9% | 73,8% |
| 143 " | 88,7% | 78,1% | 69,3% |

4

Exemple comparatif n°1

On a fait passer la même charge que dans l'exemple 1,ainsi que de la vapeur d'eau dans un rapport molaire eau/charge 1/1, sur de la silicalite non traitée à une température de 325°C sous une pression de 14,8.10[5] Pa et avec une LHSV de 41,2.

On a obtenu les résultats suivants (% en poids) :

|  |  | conversion des n-butènes | sélectivité en essences | rendement en essences |
|---|---|---|---|---|
| après | 12 heures | 91,6% | 65,2% | 59,7% |
|  | 26 heures | 87,3% | 80,7% | 70,5% |
|  | 52 heures | 60,7% | 80,4% | 48,8% |
|  | 75 heures | 20,4% | 88,6% | 18,1% |

Exemple comparatif n°2

On a fait passer la même charge que dans l'exemple 1, ainsi que de la vapeur d'eau dans un rapport molaire eau/charge de 0,51/1, sur de la silicalite non traitée à une température de 323°C sous une pression de 14,8.10[5]Pa et avec une LHSV de 31.

Après 7 heures, la conversion des n-butènes était de 70,6 % en poids et la sélectivité en essences de 87,8 %. Après 25 heures, la conversion des n-butènes n'était plus que de 6,85 %.

Exemple n°2

On a répété l'expérience décrite dans l'exemple 1, à l'exception du rapport molaire eau/charge qui a été de 0,45/1.

On a obtenu les résultats suivants (% en poids) :

|  |  | conversion des n-butènes | sélectivité en essences | rendement en essences |
|---|---|---|---|---|
| après | 10 heures | 89,2% | 80,3% | 71,6% |
|  | 24 heures | 88,6% | 84,1% | 74,5% |
|  | 54 heures | 82,0% | 81,8% | 67,1% |
|  | 78 heures | 79,1% | 81,1% | 64,2% |
|  | 97 heures | 76,2% | 79,8% | 60,8% |
|  | 122 heures | 66,5% | 80,4 | 53,5% |

Exemple n°3

On a chargé de la silicalite dans un réacteur que l'on a chauffé à 500°C pendant 3 heures sous un GHSV d'azote de 500. On a refroidi à 284°C en maintenant le débit d'azote, puis on a saturé pendant 110 minutes l'azote envoyé vers le réacteur avec du $CCl_4$.

Sur la silicalite ainsi chlorée, contenant 0,4 % en poids de chlore, on a fait passer, à une température de 340°C, sous une pression de 14,9.10[5] Pa et avec une LHSV de 30, une charge ayant la même composition que dans l'exemple 1 ainsi que de la vapeur d'eau dans un rapport molaire eau/charge de 0,7/1.

On a obtenu les résultats suivants (% en poids):

| | | conversion des n-butènes | sélectivité en essences | rendement en essences |
|---|---|---|---|---|
| après | 8 heures | 94,5% | 84,3% | 79,7% |
| | 24 heures | 92,9% | 85,3% | 79,2% |
| | 48 heures | 91,5% | 82,3% | 75,3% |
| | 75 heures | 90,8% | 83,9% | 76,2% |
| | 96 heures | 89,4% | 84,7% | 75,7% |
| | 126 heures | 89,2% | 80,2% | 71,5% |
| | 149 heures | 88,2% | 80,6% | 71,1% |
| | 157 heures | 87,4% | 80,5% | 70,4% |

Exemple 4

On a chargé dans un réacteur du TEA-silicate que l'on a chauffé à 500°C pendant 3 heures sous un GHSV d'azote de 500. On a refroidi à 278°C en maintenant le débit d'azote, puis on a saturé pendant 110 minutes l'azote envoyé vers le réacteur avec du $CCl_4$.

Sur le TEA-silicate ainsi chloré, contenant 0,4 % en poids de chlore, on a fait passer, à une température de 380°C, sous une pression de $15 .10^5 Pa$ et avec une LHSV de 30, une charge ayant la même composition que dans l'exemple 1 ainsi que de la vapeur d'eau dans un rapport molaire eau/charge de 0,7/1.

On a obtenu les résultats suivants (% poids):

| | | conversion des n-butènes | sélectivité en essences | rendement en essences |
|---|---|---|---|---|
| après | 9 heures | 93,3% | 84,4% | 78,7% |
| | 25 heures | 89,0% | 85,1% | 75,7% |
| | 59 heures | 85,6% | 85,1% | 72,8% |
| | 83 heures | 84,2% | 83,6% | 70,4% |

**Revendications**

1. Procédé de traitement catalytique de charges d'hydrocarbures, réalisé sous pression, et avec un catalyseur formé de silicalite ou de TEA-silicate préalablement stabilisés, caractérisé en ce qu'il est réalisé en présence d'eau et que le traitement de stabilisation soit une halogénation.

2. Procédé de traitement catalytique selon la revendication 1, caractérisé en ce qu'il est réalisé à une température de 250 à 550°C, sous une pression de $10^5$ à $7.10^6$ Pa, et en présence d'eau dans un rapport molaire eau/charge de 0,5 à 1,5.

3. Procédé d'aromatisation de charges d'hydrocarbures selon l'une quelconque des revendications 1 ou 2.

4. Procédé d'isomérisation de charges d'hydrocarbures selon l'une quelconque des revendications 1 ou 2.

5. Procédé d'oligomérisation d'oléfines selon l'une quelconque des revendications 1 ou 2, caractérisé en ce qu'il est réalisé à une température de 300 à 500°C, sous une pression de $2.10^5$ à $6.10^6$ Pa, en présence d'eau dans un rapport molaire eau/charge de 0,6 à 0,9, et à un LHSV de 5 à 200.

6. Procédé selon l'une quelconque des revendications 1 à 5, caractérisé en ce que la stabilisation du catalyseur consiste à :
a) halogéner ce catalyseur en le mettant en contact avec un courant gazeux comprenant
(i) un agent d'halogénation choisi dans le groupe comprenant les composés organiques aliphatiques chlorés saturés, les composés organiques aliphatiques bromés saturés, les composés organiques fluorés saturés, et leurs mélanges, ayant une tension de vapeur d'au moins 13 kPa à une température de 200 à 230°C et ayant une faible teneur en hydrogène; et
(ii) un véhiculeur gazeux non-réducteur, à une température comprise entre 200 et 500°C et pendant une période de temps suffisante pour fixer de 0,1 à 5 % en poids d'halogène sur la silicalite;
b) récupérer ou utiliser le catalyseur halogéné et stabilisé ainsi formé.

7. Procédé selon la revendication 6, caractérisé en ce que l'agent d'halogénation est choisi dans le groupe comprenant les composés organiques aliphatiques chlorés saturés, les composés organiques aliphatiques bromés saturés et leurs mélanges.

6

8. Procédé selon la revendication 7, caractérisé en ce que l'on réalise l'halogénation à une température comprise entre 250 et 300°C.

9. Procédé selon la revendication 6, caractérisé en ce que l'agent d'halogénation est choisi parmi les composés organiques aliphatiques fluorés saturés et leurs mélanges.

10. Procédé selon la revendication 9, caractérisé en ce que l'on réalise l'halogénation à une température comprise entre 450 et 500°C .

11. Procédé selon l'une des revendications 6 à 10, caractérisé en ce que l'agent d'halogénation a une tension de vapeur supérieure a 40 kPa à une température de 200 à 230°C.

12. Procédé selon la revendication 11, caractérisé en ce que l'agent d'halogénation a une tension de vapeur comprise entre 40 et 53 kPa à une température de 200 à 230°C.

13. Procédé selon l'une des revendications 6 à 12, caractérisé en ce que l'agent d'halogénation est choisi parmi les composés ayant un rapport atomique halogène/carbone égal ou supérieur à 1, et leurs mélanges.

14. Procédé selon l'une des revendications 6 à 13, caractérisé en ce que l'agent d'halogénation est choisi parmi les paraffines halogénées contenant de 1 à 4 atomes de carbone, et leurs mélanges.

15. Procédé selon l'une des revendications 6 à 14, caractérisé en ce que l'agent d'halogénation est choisi dans le groupe comprenant le tétrachlorure, le tétrabromure et le tétrafluorure de carbone, le chloroforme, le bromoforme, le fluoroforme, l'hexafluoroéthane, le pentachloroéthane, et le difluorométhane.

16. Procédé selon l'une des revendications 6 à 13, caractérisé en ce que l'agent d'halogénation est choisi parmi les éthers halogénés contenant de 2 à 4 atomes de carbone, et leurs mélanges.

17. Procédé selon la revendication 11, caractérisé en ce que l'agent halogénant est choisi parmi le dipentachloroéthyl éther, le di-tri-chlorométhyl éther, leurs homologues bromés, et leurs mélanges.

18. Procédé selon l'une quelconque des revendications 6 à 17, caractérisé en ce que le véhiculeur gazeux non-réducteur est choisi parmi l'azote, le dioxyde de carbone, l'oxygène et leurs mélanges.

19. Procédé selon l'une quelconque des revendications 6 à 18, caractérisé en ce que la mise en contact a lieu pendant une période de temps suffisante pour fixer de 0,1 à 1 % en poids d'halogène sur la silicalite ou sur le TEA-silicate.

## Patentansprüche

1. Verfahren zur katalytischen Behandlung von Kohlenwasserstoff-Ausgangsmaterialien mit einem aus vorher stabilisiertem Silicalit oder TEA-Silikat gebildeten Katalysator, dadurch gekennzeichnet, daß es in Gegenwart von Wasser durchgeführt wird und die Stabilisierungsbehandlung in einer Halogenierung besteht.

2. Verfahren zur katalytischen Behandlung nach Anspruch 1, dadurch gekennzeichnet, daß es bei einer Temperatur von 250 bis 550°C, unter einem Druck von $10^5$ bis $7 \times 10^6$ Pa und in Gegenwart von Wasser in einem Molverhältnis von Wasser/Ausgangsmaterial von 0,5 bis 1,5 durchgeführt wird.

3. Verfahren zur Aromatisierung von Kohlenwasserstoff-Ausgangsmaterialien nach einem der Ansprüche 1 oder 2.

4. Verfahren zur Isomerisierung von Kohlenwasserstoff-Ausgangsmaterialien nach einem der Ansprüche 1 oder 2.

5. Verfahren zur Oligomerisierung von Olefinen nach einem der Ansprüche 1 oder 2, dadurch gekennzeichnet, daß es bei einer Temperatur von 300 bis 500°C, einem Druck von $2 \times 10^5$ bis $6 \times 10^6$ in Gegenwart von Wasser in einem Molverhältnis von Wasser/Beschickungsmaterial von 0,6 bis 0,9 und bei einem LHSV-Wert von 5 bis 200 durchgeführt wird.

6. Verfahren nach einem der Ansprüche 1 bis 5, dadurch gekennzeichnet, daß die Stabilisierung des Katalysators aus folgenden Schritten besteht:

a) Halogenieren des Katalysators, indem man ihn mit einem Gasstrom in Kontakt bringt, der folgende Bestandteile enthält:

(i) ein Halogenierungsmittel, das aus der Gruppe organische, aliphatische, chlorierte, gesättigte Verbindungen, organische, aliphatische, bromierte, gesättigte Verbindungen, organische, fluorierte, gesättigte Verbindungen und deren Gemische ausgewählt ist, einen Dampfdruck bei einer Temperatur von 200 bis 230°C von mindestens 13 kpa aufweist und einen geringen Wasserstoffgehalt besitzt; und

(ii) einen nicht-reduzierenden, gasförmigen Träger, bei einer Temperatur zwischen 200 und 500°C und während einer Zeitspanne die ausreicht, um 0,1 bis 5 Gew.-%Halogen am Silicalit zu fixieren;

b) Gewinnen oder Verwenden des auf diese Weise gebildeten halogenierten und stabilisierten Katalysators.

7. Verfahren nach Anspruch 6, dadurch gekennzeichnet, daß das Halogenierungsmittel aus der Gruppe organische, aliphatische, chlorierte, gesättigte Verbindungen, organische, aliphatische, bromierte, gesättigte Verbindungen und deren Gemische ausgewählt ist.

8. Verfahren nach Anspruch 7, dadurch gekennzeichnet, daß die Halogenierung bei einer Temperatur zwischen 250 und 300°C durchgeführt wird.

9. Verfahren nach Anspruch 6, dadurch gekennzeichnet, daß das Halogenierungsmittel unter organischen, aliphatischen, fluorierten, gesättigten Verbindungen und deren Gemischen ausgewählt wird.

10. Verfahren nach Anspruch 9, dadurch gekennzeichnet, daß man die Halogenierung bei einer Temperatur zwischen 450 und 500°C durchführt.

11. Verfahren nach einem der Ansprüche 6 bis 10, dadurch gekennzeichnet, daß das Halogenierungsmittel bei einer Temperatur von 200 bis 230°C einen Dampfdruck von mehr als 40 kPa aufweist.

12. Verfahren nach Anspruch 11, dadruch gekennzeichnet, daß das Halogenierungsmittel bei einer Temperatur von 200 bis 230°C einen Dampfdruck zwischen 40 und 53 kPa aufweist.

13. Verfahren nach einem der Ansprüche 6 bis 12, dadurch gekennzeichnet, daß das Halogenierungsmittel unter Verbindungen mit einem Atomverhältnis Halogen/Kohlenstoff vom 1 oder darüber und deren Gemischen ausgewählt wird.

14. Verfahren nach einem der Ansprüche 6 bis 13, daß das Halogenierungsmittel unter halogenierten Paraffinen mit 1 bis 4 Kohlenstoffatomen und deren Gemischen ausgewählt wird.

15. Verfahren nach einem der Ansprüche 6 bis 14, dadurch gekennzeichnet, daß das Halogenierungsmittel aus der Gruppe Tetrachlor-, Tetrabrom- und Tetrafluorkohlenstoff, Chloroform, Bromoform, Bluoroform, Hexafluoroethan, Pentachlorethan und Difluormethan ausgewählt wird.

16. Verfahren nach einem der Ansprüche 6 bis 13, dadurch gekennzeichnet, daß das Halogenierungsmittel unter halogenierten Ethern mit einem Gehalt an 2 bis 4 Kohlenstoffatomen und deren Gemischen ausgewählt wird.

17. Verfahren nach Anspruch 11, dadurch gekennzeichnet, daß das Halogenierungsmittel unter Dipentachlorethylether, Ditrichlormethylether, deren bromierten Homologen und deren Gemischen ausgewählt wird.

18. Verfahren nach einem der Ansprüche 6 bis 17, dadurch gekennzeichnet, daß der nicht-reduzierende, gasförmige Träger unter Stickstoff, Kohlendioxid, Sauerstoff und deren Gemischen ausgewählt wird.

19. Verfahren nach einem der Ansprüche 6 bis 18, dadurch gekennzeichnet, daß der Kontakt während einer Zeitdauer erfolgt, die ausreicht, um 0,1 bis 1 Gew.-% Halogen auf dem Silicalit oder dem TEA-Silikat zu fixieren.

## Claims

1. Catalytic treatment process of hydrocarbon feedstocks which consists in converting said hydrocarbon feedstocks under pressure with a catalyst selected from either silicates or TEA-silicate which have previously been stabilized, said process being characterized by the presence of water and by the particular stabilization treatment by halogenation.

2. The process of Claim 1 characterized in that it is carried out at a temperature from about 270°C to about 550°C, under a pressure comprised between $10^5$ and $7 \times 10^6$ Pa, and in the presence of water in a molar ratio water/feed comprised between about 0.5 and about 1.5.

3. Process according to either of Claims 1 or 2 wherein the hydrocarbon feedstocks are aromatized.

4. Process according to either of Claims 1 or 2 wherein the hydrocarbon feedstocks are isomerized.

5. Process according to either of Claims 1 or 2 wherein the olefinic hydrocarbon feedstocks are oligomerized at a temperature comprised between 300°C and 500°C under a pressure comprised between $2 \times 10^5$ and $6 \times 10^6$ Pa, in the presence of water in a molar ratio water/feed of 0.6 to 0.9 and at a LHSV of from about 5 to about 200.

6. A process according to any one of Claims 1 to 5 wherein the stabilization of the catalyst comprises:

(a) halogenating said catalyst by contacting it with a gaseous stream comprising:

(ii) an halogenation agent selected from the group comprising the organic saturated aliphatic chlorinated compounds, the organic saturated aliphatic brominated compounds, the organic saturated aliphatic fluorinated compounds and mixtures thereof, having a vapor pressure of at least 13 kPa at a temperature of 200 to 230°C and having a low hydrogen content; and

(ii) non-reducing gaseous vehicle, at a temperature comprised between 200 and 500°C and during a period of time sufficient to fix from 0.1 to 5% by weight of halogen on the catalyst;

(b) recovering or using the so-formed stabilized and halogenated catalyst.

7. A process according to Claim 6 wherein the halogenation agent is selected from the group comprising the organic saturated chlorinated aliphatic compounds, the organic saturated brominated aliphatic compounds and mixtures thereof.

8. A process according to Claim 7 wherein the halogenation is carried out a temperature comprised between about 250 and 300°C.

9. A process according to Claim 8 wherein the halogenation agent is selected from the group of the organic saturated fluorinated aliphatic compounds and mixtures thereof.

10. A process according to Claim 9 wherein the halogenation is carried out at a temperature comprised between about 450 and 500°C.

11. A process according to any one of Claims 6 to 10 wherein the halogenation agent has a vapor pressure higher than 40 kPa at a temperature comprised between 200 and 230°C.

12. A process according to Claim 11 wherein the halogenation agent has a vapor pressure comprised between about 40 and about 53 kPa at a temperature comprised between 200 and 230°C.

13. A process according to any one of Claims 6 to 12 wherein the halogenation agent is selected from the compounds having an halogen/carbon atomic ratio equal to or higher than 1, and mixtures of these compounds.

14. A process according to any one of Claims 6 to 13 wherein the halogenation agent is selected from the halogenated paraffins having from 1 to 4 carbon atoms, and mixtures thereof.

15. A process according to any one of Claims 6 to 14 wherein the halogenation agent is selected from the group comprising carbon tetrachloride, carbon tetrabromide, carbon tetrafluoride, chloroform, bromoform, fluoroform, hexafluoroethane, pentachloroethane and difluoromethane.

16. A process according to any one of Claims 6 to 13 wherein the halogenation agent is selected from the halogenated ethers containing from 2 to 4 carbon atoms, and mixtures thereof.

17. A process according to Claim 11 wherein the halogenation agent is selected from the group comprising di-pentachloroethyl ether, di-tri-chloromethyl ether, their brominated homologs and mixtures thereof.

18. A process according to any one of Claims 6 to 17 wherein the non-reducing gaseous vehicle is selected from the group comprising nitrogen, carbon dioxide, oxygen and mixtures thereof.

19. A process according to any one of Claims 6 to 18 wherein the contact is carried out during a period of time sufficient to fix from 0.1 to 1% by weight of halogen on the silicalite or the TEA-silicate.